# EUROPEAN PATENT SPECIFICATION

(11) **EP 1 713 346 B1**
(45) Date of publication and mention of the grant of the patent: **26.12.2012**
(21) Application number: 04731506.4
(22) Date of filing: 06.05.2004
(51) Int. Cl.: A23F 3/00

(54) **NATURAL TEA HAVING EFFECT OF LIVER FUNCTION RECOVERY AND METHOD FOR PREPARING THE SAME**
NATÜRLICHER TEE MIT LEBERFUNKTIONSGESUNDUNGSWIRKUNG SOWIE VERFAHREN ZU SEINER HERSTELLUNG
THE NATUREL DESTINE A RELANCER LA FONCTION HEPATIQUE ET METHODE DE PREPARATION

(30) Priority: 29.01.2004 KR 2004005848
(43) Date of publication of application: 25.10.2006
(73) Proprietor: Nam, Jong-Hyun, Seoul 138-110 (KR)
(72) Inventor: Nam, Jong-Hyun, Seoul 138-110 (KR)
(74) Representative: Ellis, Katherine Elizabeth Sleigh
(86) International application number: PCT/KR2004/001050
(87) International publication number: WO 2005/072532

(56) References cited:
- WO-A1-00/69452
- JP-A- 6 327 406
- KR-A- 950 016 538
- KR-A- 20010 069 898
- KR-A- 20010 109 708
- KR-A- 20030 079 289
- DATABASE WPI Week 199934 Thomson Scientific, London, GB; AN 1999-403620 XP002561959 & RO 114 297 B1 (DOBOS A) 30 March 1999 (1999-03-30)
- DATABASE WPI Week 199624 Thomson Scientific, London, GB; AN 1996-232827 XP002561960 & HU 69 404 A (SZEVIKI SZERVES VEGYIPARI RT K) 28 September 1995 (1995-09-28)

## Description

### BACKGROUND OF THE INVENTION

### Field of the Invention

The present invention relates to a type of natural tea having the effect of revitalizing liver functions, and a method for preparing the same. More particularly, the present invention relates to a natural tea having the effects of revitalizing liver functions, which contains an extract from *Robinia pseudo-acacia* as a main active ingredient, which is obtained under optimal extraction conditions, as well as a method for preparing the same.

### Background Art

Generally, alcoholic drinks have appeared from the beginning of recorded human history and have been favored drinks until now. However, such alcoholic drinks can cause various problems, and among such problems, health problems caused by drinking alcohol are those most frequently pointed out. Namely, drinking alcohol is known to cause problems such as liver disease and reductions in stomach, colon and brain functions, and the appearance of such damage from alcohol have continued until the present time. Thus, research undertaken to protect the liver from alcohol and to relieve hangovers caused by drinking have been continuously conducted.

As used herein, the term "hangover" refers to various symptoms such as unpleasantness, headache and decrease in mental and physical ability to work, which occur when becoming sober and may last until the next day or even two days later after drinking alcohol. Such hangovers seem to occur because alcohol or acetaldehyde is accumulated in the human body due to lack of alcohol dehydrogenase or acetaldehyde dehydrogenase. In other words, when alcohol is ingested into the body via drinking, it is degraded by alcohol dehydrogenase while it is first converted into acetaldehyde and then into acetic acid, a metabolite thereof. If this metabolism does not occur smoothly, the accumulated ethyl alcohol or the acetaldehyde converted during the alcohol degradation process will lead to toxin processing, causing damages to liver and brain cells, so that metabolic disorders in the body will occur, causing whole body fatigue, abdominal distension, emesis, headache, and even chill and abdominal pain, resulting in hangovers.

Such hangovers make daily life abnormal and cause lack of energy, having severe effects not only upon the hangover person himself but also upon other persons. Thus, such hangovers incur significantly negative results in daily life.

From olden times, in folk remedies for relief of such hangovers, various foods have been used. Namely, foods, such as ox-blood soup, bean sprout soup, dried Pollack soup, shellfish soup, oysters, or juices such as that of *Aegopodium podagraria* L, radishes, cucumbers, leeks, spinach, lotus roots, arrowroots, pine needle juice, ginseng juice, and green tea leaves, have been believed to be effective in relieving hangovers. Particularly, green tea leaves have been thought to be greatly effective in relieving hangovers, since they contain polyphenol and thus can easily decompose acetaldehyde.

Furthermore, drink-type beverages for relieving such hangovers have been developed and sold. For example, Korean patent No. 0181168 discloses a natural tea for relieving hangovers and a method for preparing the same. The natural tea prepared according to this Korean patent contains an extract from, or a powder of the leaf, stem or root of *Alnus japonica* and *Sorbus commixta* as an active component, as well as given amounts of extracts from the fruit of *Ligustrum japonicum Thunberg* and the root of *Pueraria.* Also, Korean patent application laid-open No. 2001-019767 discloses natural tea for relieving hangovers and a method for preparing the same. The natural tea prepared according to this Korean patent publication contains Chinese medicinal materials, including *Pueraria* flowers, *Pueraria* roots, *Liquordice* roots, *Atractylodes Rhizome White,* dried orange peels, *Alismatis Rhizoma, Lycium chinense,* and ginger.

Korean patent application laid open No. 2001-109708 discloses natural tea having a controlling effect on liver function, containing extracts of Cassiae and Hedyotis diffusa Willd plants.

By such various methods, various drinks for relieving hangovers, each having a unique characteristic, have been developed.

However, in spite of such various efforts to relieve hangovers, there is still a need for the development of drinks or foods that can effectively relieve hangovers.

### SUMMARY OF THE INVENTION

Accordingaly, an object of the present invention is to provide a natural tea, having excellent effects in revitalizing liver functions by rapidly lowering blood alcohol concentration when taken before or after drinking alcohol, and which allows the human body to be maintained at normal conditions before or after generation of a hangover, thereby providing health benefits.

Still another object of the present invention is to provide a method for preparing the natural tea according to the above object.

According to a first aspect of the present invention, there is provided a natural tea as specified in claim 1.

It will be understood that the extract from *Robinia psueudo-acacia,* which is used in the present invention, includes all parts of *Robinia pseudo-acacia,* including the leaf, stem, flower, root and fruit of *Robinia pseudo-acacia.*

In the natural tea according to another embodiment of the present invention, the extract from the leaf, stem, flower and root of Robinia pseudo-acacia is contained as a main active component, to which at least one selected from *Hedyotis diffusa,* Amomi *semen, Glycyrrhiza* and Pueraiae Flos extracts can be added as a minor active component. However, the present invention is achieved by the main active component, and such minor active components are optional and not critical to the present invention.

In another embodiment, the present invention provides natural tea having the effect of revitalizing liver functions, which comprises a mixture of 10-80% by weight of an extract from *Robinia* pseudo-acacia and 20-90% by weight of *Hedyotis diffusa.*

In still another embodiment, the present invention provides a natural tea having the effect of revitalizing liver functions, which comprises a mixture of 10-65% by weight of an extract from *Robinia pseudo-acacia,* 20-80% by weight of *Hedyotis diffusa,* 10-60% by weight of *Amomi semen,* and 10-50% by weight of *Glycyrrhiza.*

According to a second aspect of the present invention, there is provided a method for preparing natural tea as specified in claim 6.

In the method according to the present invention, the extraction step is conducted at a temperature of 50-150 °C. If the extraction temperature is below 50 °C, the active ingredients of *Robinia pseudo-acacia* will not be easily extracted. If the extraction temperature is above 150 °C, an extraction effect will not be increased and the active ingredients can be modified. The extraction step is most preferably conducted at a temperature of 50-90 °C.

In another embodiment of the present invention, the extract is centrifuged at 2,500-7,500 rpm to separate robinine.

In still another embodiment of the present invention, each component is dried in the shade and pulverized to a size of 50-100 meshes such that powder tea is provided.

Acacia, which is used as a main component in the present invention, is an evergreen belonging to the family *Leguminosae* of the order *Rosales,* dicotyledonous plants. About 500 species of acacia are found in the tropical and temperate zones of the world, particularly in Australia. Its leaves are double paripinnate compound leaves, each having very small leaflets and a peptiole that is flat or similar to a leaf. Also, its flowers have a yellow or white color, and an acacia often designated as *Robinia pseudo-*acacia is also native to North America.

*Hedyotis diffusa,* which is used in the present invention, is an annual herb belonging to the family *Rubiaceae* and grows to a height of around 30 cm. It is native to China, but is also found on Mt. Halla in island of Jejudo and on the swampy land of Baekun mountain in Jeolla-Namdo in Republic of Korea. It can be regarded as a recently discovered medicinal herb not described in olden Chinese medicinal books, such as Compendium of Materia Medica. It has been used in research and experiments in China since 40 years ago, when it was first described in the Guangxi Journal of Traditional Chinese Medicine just after the year 1945. *Hedyotis diffusa* has been widely known in the world, since its efficacy and action were described in detail in the book "Traditional Chinese Medicine Research" published by the Taiwan Center of Traditional Chinese Medicine.

*Glycyrrhiza,* which is used in the present invention, is a perennial plant belonging to the family *Leguminosae* of the order *Rosales,* which are dicotyledonous plants. Its root has a reddish brown color and is deeply penetrated into the ground. Its stem is angular and grows straight to a height of around 1 m. The stem has white fuzz closely distributed thereon and displays a light ash color. Also, the stem has pellucid dots spread thereupon. The leaf is an alternate, imparipinnate compound leaf with 1-17 leaflets, which are egg-like in outline and have a sharp end. Each of the leaflets is 2-5 cm long and 1-3 cm wide, has white fuzz and pellucid dots on both sides, and has no serrate. Examples of *Glycyrrhiza* includes G. *glabra var. glandulifera,* native to Siberia, and G. *glabra,* native to Spain. A similar species includes G. *pallidiflora.*

*Pueraiae Flos,* which is used in the present invention, refers to the *Pueraria* flower in Chinese medicine and has been mainly used as a drug for relieving alcoholic poisoning and for stopping the discharging of blood.

The inventive natural tea prepared as described above has the effects of relieving hangovers and revitalizing liver functions. This natural tea comprises either an extract from, or a powder of the leaf, stem, flower and root of *Robinia pseudo-acacia* distributed in the Orient, as a main active component, to which at least one selected from *Hedyotis diffusa, Amomi semen, Glycyrrhiza,* and *Pueraiae Flos* extracts, which act to neutralize poison, is added at a given amount. When taken before or after drinking alcohol, the inventive natural tea shows the excellent effects of relieving hangovers and revitalizing liver functions.

### BRIEF DESCRIPTION OF THE DRAWINGS

FIG. 1 is a graphic diagram showing a change in body weight of the experimental what was used during a test period;
FIG. 2 is a graphic diagram showing an effect of long-term alcohol administration on hematocrit content; and
FIG.3 is a graphic diagram showing an effect of long-term alcohol administration on hemoglobin content.

### DETAILED DESCRIPTION OF THE PREFERRED EMBODIMENTS

The present invention will hereinafter be described in further detail by examples and test examples. It should however be borne in mind that the present invention is not limited to or by the examples.

### Example 1: Preparation of acacia extract

The leaf, stem, flower and root of *Robinia pseudo-acacia* were screened, and washed with water. A given amount of the water-washed material was extracted with alcohol at about 70 °C for 5 hours, to give acacia extract.

### Example 2

The acacia extract prepared according to Example 1 was used as a main active component, to which conventional additives, such as a sweetening agent, spices and a coloring agent, were added, thereby preparing natural tea having the effects of relieving hangovers and revitalizing liver functions.

### Example 3

Natural tea having the effects of relieving hangovers and revitalizing liver functions was prepared in the same manner as in Example 2, except that a mixture of 80% by weight of the acacia extract and 20% by weight of an extract from *Hedyotis diffusa* was used.

### Example 4

Natural tea having the effects of relieving hangovers and revitalizing liver functions was prepared in the same manner as in Example 2, except that a mixture of 70% by weight of the acacia extract and 30% by weight of an extract from *Hedyotis diffusa* was used.

### Example 5

Natural tea having the effects of relieving hangovers and revitalizing liver functions was prepared in the same manner as in Example 2, except that a mixture of 60% by weight of the acacia extract and 40% by weight of an extract from *Hedyotis diffusa* was used.

### Example 6

Natural tea having the effects of relieving hangovers and revitalizing liver functions was prepared in the same manner as in Example 2, except that a mixture of 50% by weight of the acacia extract and 50% by weight of an extract from *Hedyotis diffusa* was used.

### Example 7

Natural tea having the effects of relieving hangovers and revitalizing liver functions was prepared in the same manner as in Example 2, except that a mixture of 40% by weight of the acacia extract and 60% by weight of an extract from *Hedyotis diffusa* was used.

### Example 8

Natural tea having the effects of relieving hangovers and revitalizing liver functions was prepared in the same manner as in Example 2, except that a mixture of 30% by weight of the acacia extract and 70% by weight of an extract from *Hedyotis diffusa* was used.

### Example 9

Natural tea having the effects of relieving hangovers and revitalizing liver functions was prepared in the same manner as in Example 2, except that a mixture of 20% by weight of the acacia extract and 80% by weight of an extract from *Hedyotis diffusa* was used.

### Example 10

Natural tea having the effects of relieving hangovers and revitalizing liver functions was prepared in the same manner as in Example 2, except that a mixture of 10% by weight of the acacia extract and 90% by weight of an extract from *Hedyotis diffusa* was used.

### Examples 11-36

Natural teas having the effects of relieving hangovers and revitalizing liver functions were prepared in the same manner as in Example 2, except that extracts from *Robinia pseudo-acacia, Hedyotis diffusa, Amomi semen, Glycyrrhiza,* and/or *Pueraiae Flos* were mixed with each other at a mixing ratio given in Table 1 below.

**Table 1**

| Table 1 | | | | (unit:wt%) | |
|---|---|---|---|---|---|
| | Acacia extract | *Hedyotis diffusa* extract | *Amomi semen* extract | *Glycyrrhiza* extract | *Pueraiae Flos* extract |
| Example 11 | 60 | 20 | 10 | 10 | 0 |
| Example 12 | 50 | 30 | 10 | 10 | 0 |
| Example 13 | 50 | 20 | 20 | 10 | 0 |
| Example 14 | 40 | 40 | 10 | 10 | 0 |
| Example 15 | 40 | 30 | 20 | 10 | 0 |
| Example 16 | 30 | 50 | 10 | 10 | 0 |
| Example 17 | 30 | 40 | 20 | 10 | 0 |
| Example 18 | 30 | 40 | 10 | 20 | 0 |
| Example 19 | 30 | 30 | 20 | 10 | 10 |
| Example 20 | 20 | 40 | 10 | 20 | 10 |
| Example 21 | 20 | 40 | 10 | 10 | 20 |
| Example 22 | 20 | 50 | 10 | 10 | 10 |
| Example 23 | 20 | 40 | 20 | 10 | 10 |
| Example 24 | 20 | 30 | 10 | 20 | 20 |
| Example 25 | 20 | 20 | 10 | 20 | 30 |
| Example 26 | 10 | 80 | 10 | 0 | 0 |
| Example 27 | 10 | 70 | 10 | 10 | 0 |
| Example 28 | 10 | 60 | 10 | 10 | 10 |
| Example 29 | 10 | 50 | 20 | 10 | 10 |
| Example 30 | 10 | 40 | 20 | 20 | 10 |
| Example 31 | 10 | 30 | 30 | 20 | 10 |
| Example 32 | 10 | 20 | 40 | 20 | 10 |
| Example 33 | 10 | 20 | 50 | 20 | 10 |
| Example 34 | 10 | 20 | 60 | 0 | 10 |
| Example 35 | 10 | 20 | 0 | 50 | 20 |
| Example 36 | 10 | 20 | 0 | 20 | 50 |

### Test example 1: Change in body weight

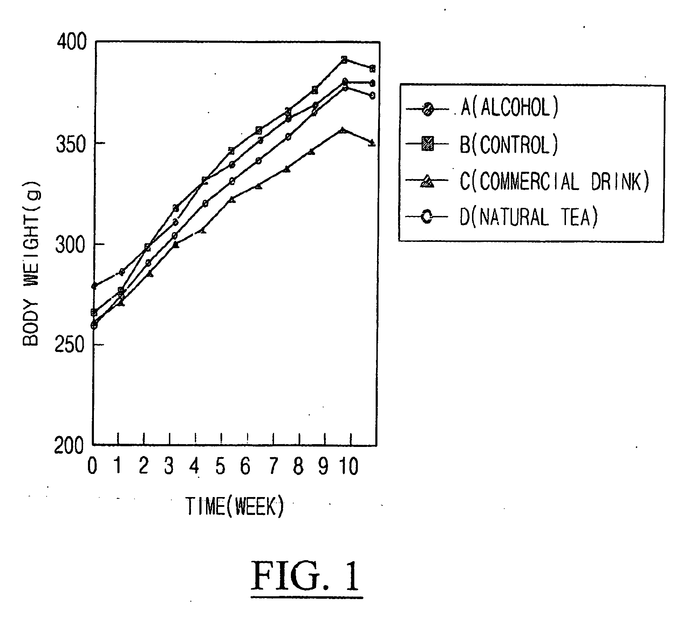

FIG. 1 shows a change in body weight during a test period. Up to seven weeks after test start, three test groups except for the control group B were administered only with ethanol in order to cause liver damage.

Upon test start, body weights were 278.9 ± 13.7 g, 261.6 ± 22.0 g, 260.6 ± 23.6 g and 263.7 ± 13.1 g for A, B, C and D groups, respectively. The body weight of the groups administered with alcohol was about 17 g higher than the control group.

The control group B, which was not administered with alcohol and drinks, showed the highest increase in body weight.

In spite of long-term ethanol administration, the alcohol-only group A showed a continuous increase in body weight during a test period as compared to the other two groups C and D administered with hangover relief drinks. Presumably, this is because the body weight of the group A upon the start of tests was higher than the other two groups C and D. The hangover relief drink-administered groups showed an increase in body weight up to three weeks after test start without a significant difference between the groups. However, after three weeks, group D, the group administered with the inventive natural tea, showed a clear increase in body weight as compared to the group C administered with usual hangover relief drinks in the market.

### Example 2: Hematocrit and hemoglobin contents

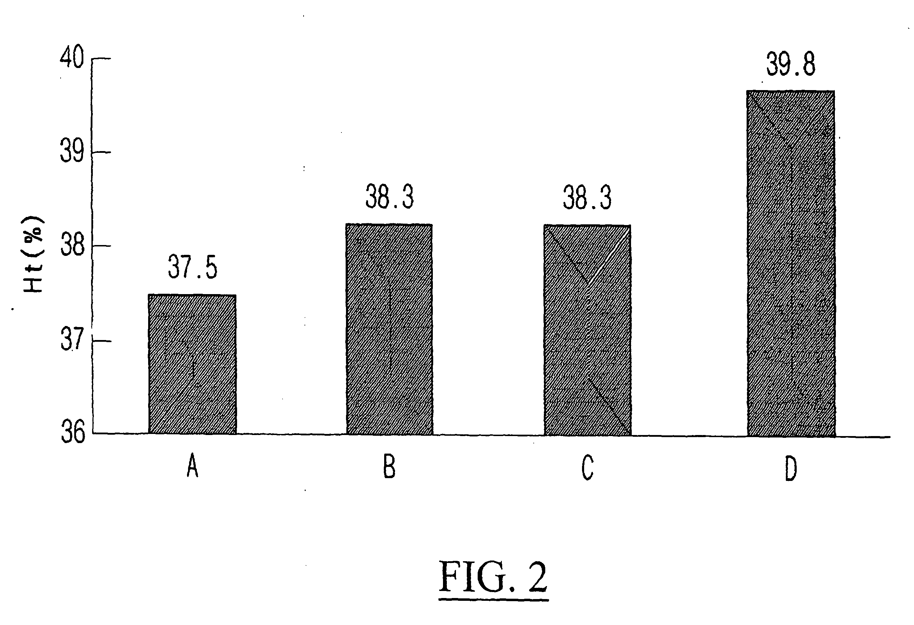

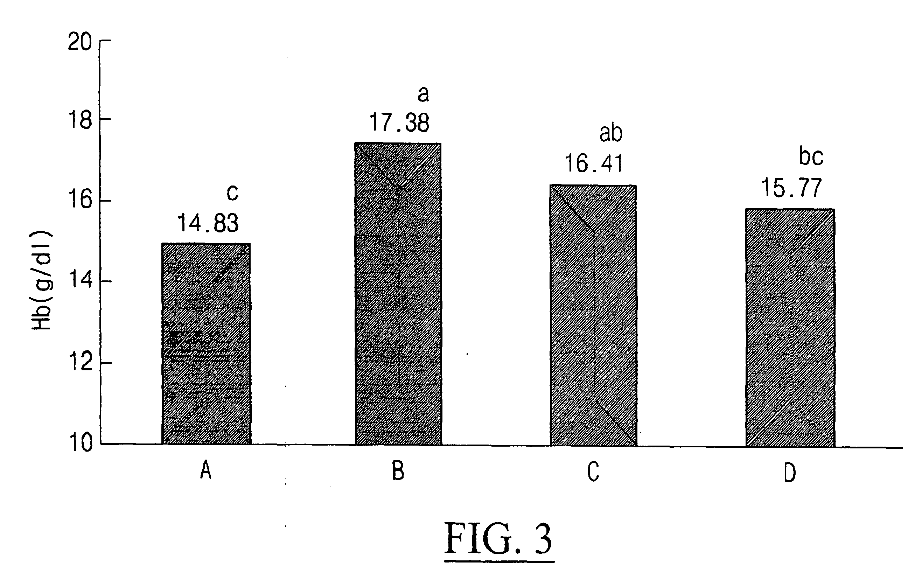

FIG. 2 and 3 show hematocrit (Ht) and hemoglobin (Hb) contents, respectively, which were measured upon the completion of the testing. There was no significant difference in Ht content between the treated groups. However, group D, the group administered with the inventive natural tea, showed the highest Ht content of 39.8%. The control group B and group C, the group administered with a usual hangover relief drink in the market, showed an Ht content of 38.0%, and group A, the group only administered with alcohol, showed a somewhat lower Ht content of 37.5%.

Hb contents were significantly different statistically between the treated groups (p<0.05). The control group showed the highest Hb content of 17.36 g/dl, the commercial hangover relief drink-administered group C showed 163.41 g/dl, the inventive natural tea-administered group D showed 15.77 g/dl, and the alcohol-only group A showed 14.83 g/dl.

### Example 3: Serum lipid content

Table 2 below shows serum lipid content measured upon the end of the test. The control group B showed the highest triglyceride (TG) content of 76.0 mg/dl. The commercial hangover relief drink-administered group C and the inventive natural tea-administered group D showed substantially similar TG contents of 53.6 mg/dl and 49.0 mg/dl, respectively.

Total cholesterol (TC) contents were in a range of 121.2-127.6 mg/dl without the difference between the treated groups. Regarding high-density lipoprotein cholesterol (HDL) contents, the inventive natural tea-administered group D showed the highest value of 39.6 mg/dl, and the commercial drink-administered group C showed the lowest value of 31.3 mg/ml (p<0.05).

Low-density lipoprotein cholesterol (LDL) contents were not different between the test groups, but the commercial drink product-administered group showed a somewhat higher value of 80.2 mg/dl. Atherogenic index (AI), which is a risk factor for adult diseases, was 2.14 in the inventive natural tea-administered group D, which is significantly lower statistically than the other treated groups (p<0.05).

Table 2: Effect of long-term alcohol administration on serum lipid concentration (mean ± SD)

| Treated groups (n=9) | Serum lipid (mg/dl) | | | | |
|---|---|---|---|---|---|
| | TG | TC | HDL | LDL | Atherogenic index |
| A | 58.5 ± 17.0 | 121.2 ± 7.9 | 33.3 ± 5.6 | 76.4 ± 8.9 | 2.75 ± 0.60 |
| B | 76.0 ± 30.0 | 127.6 ± 11.6 | 37.2 ± 6.9 | 75.2 ± 6.1 | 2.48 ± 0.37 |
| C | 53.6 ± 12.3 | 122.2 ± 7.6 | 31.3 ± 7.0 | 80.2 ± 9.4 | 3.10 ± 10.3 |
| D | 49.0 ± 16.7 | 123.5 ± 7.8 | 39.6 ± 4.0 | 74.1 ± 9.9 | 2.14 ± 0.32 |

| | | | | | |
|---|---|---|---|---|---|
| * TG: triglyceride TC: total cholesterol HDL: high-density lipoprotein cholesterol LDL: low-density lipoprotein cholesterol Atherogenic index = (TC-HDL)/HDL Superscripts indicated with different letters have shown significant difference between mean values (p<0.05). | | | | | |

### Test example 4: Liver function examination

Table 3 below shows the effect of long-term alcohol administration on liver functions. Bilirubin, ALP, GOT and r-GTP contents were statistically different between the treated groups (p<0.05). The bilirubin contents were 3.54 mg/dl, the highest value, in the control group B, and 1.21 mg/dl, the lowest value, in the inventive natural tea-administered group D. Alkaline phosphatase (ALP) activities were 17.7 K-A unit, the highest value, in the alcohol-only group A, and 11.9 K-A unit, the lowest value, in the natural tea-administered group D. Glutamic oxaloacetic transaminase (GOT) activities were 77.4 IU/L, the highest value, in the alcohol-only group A, and 60.7 IU/L, the lowest value, in the natural tea-administered group D. GPT activities were not significantly different between the treated groups but were somewhat lower in the inventive natural tea-administered group D. y-glutamyl transpeptidase (γ-GTP) contents which are used to evaluate alcoholic fatty liver were 127.6 mU/ml, the highest value, in the commercial hangover relief drink-administered group C, and 122.6 mU/ml, the lowest value, in the alcohol-only group A, which is statistically lower than the commercial product-administered group C (p<0.05). Meanwhile, lactate dehydrogenase (LDH) activities were not significantly different between the treated groups. The control group B showed the highest LDH activity of 398.0 µ/L, and the inventive natural tea-administered group D showed the lowest LDH activity of 339.7 µ/L.

**Table 3: Effect of long-term alcohol administration on liver functions**

| Treated groups (n=9) | Bilirubin (mg/dl) | ALP (K-A unit) | GOT | GPT | r-GTP (mU/ml) | LDH (µ/L) |
|---|---|---|---|---|---|---|
| | | | (IU/L) | | | |
| A | 1.42 ± 1.02 | 17.7 ± 2.01 | 77.4 ± 16.6 | 37.4 ± 9.9 | 122.6 ± 54.7 | 375.3 ± 62.0 |
| B | 3.54 ± 2.01 | 13.8 ± 4.83 | 68.8 ± 7.3 | 34.6 ± 7.3 | 77.0 ± 34.4 | 398.0 ± 57.4 |
| C | 2.35 ± 1.05 | 15.4 ± 3.64 | 74.0 ± 9.8 | 37.3 ± 6.4 | 127.6 ± 48.5 | 353.9 ± 58.4 |
| D | 1.21 ± 0.63 | 11.9 ± 2.59 | 60.7 ± 10.1 | 31.8 ± 5.5 | 115.5 ± 41.7 | 339.7 ± 76.5 |

| | | | | | | |
|---|---|---|---|---|---|---|
| ALP: alkaline phosphatase (*K-A unit: King-Armstrong unit) GOT: glutamic oxaloacetic transaminase GPT: glutamic pyruvic transaminase γ-GTP: r-glutamyl transpeptidase LDH: lactate dehydrogenase Superscripts indicated with different letters have shown significant difference between mean values (p<0.05). | | | | | | |

### Test example 5: Blood ethanol and acetaldehyde concentrations, and alcohol dehydrogenase (ADH) activity

As given in Table 4 below, total alcohol intakes were zero in the control group B with no administration of alcohol and hangover relief drinks. Total alcohol intakes were 28.4 g in the alcohol-only group A, 26.4 g in the commercial hangover relief drink-administered group C, and 27.1 g in the inventive natural tea-administered group D. Thus, the total alcohol intakes were statistically different between the ethanol-treated groups (p<0.05).

Blood ethanol concentrations, which had been measured after long-term alcohol administration, were 0.015%, 0.010%, 0.004% and 0.003% as averages in the commercial hangover relief drink-administered group C, the alcohol-only group A, the control group B and the inventive natural tea-administered group D, respectively (p<0.05). Meanwhile, blood acetaldehyde concentrations were 82.6 ± 36.2 µg/dl, 39.8 ± 44.0 µg/dl, and 32.2 ± 35.6 µg/dl in the commercial hangover relief drink-administered group, the inventive natural tea-administered group and the control group, respectively. Thus, the blood acetaldehyde concentration in the inventive natural tea-administered group was lower than the commercial hangover relief drink-administered group but similar to the control group. Also, the blood acetaldehyde concentration in the alcohol-only group A was 222.3 ± 85.8 µg/dl which is much higher than the other three groups.

Alcohol dehydrogenase (ADH) activity was 17.98 nmol NADH/min/mg protein, the highest value, in the inventive natural tea-administered group D. ADH activities were 16.8 nmol NADH/min/mg protein in the commercial hangover relief drink-administered group C, 15.55 nmol NADH/min/mg protein in the control group B, and 15.00 nmol NADH/min/mg protein in the alcohol-only group D. Thus, the inventive natural tea-administered group D showed a somewhat higher ADH activity than the other three groups.

**Table 4: Alcohol intake during test period, blood alcohol and acetaldehyde concentrations, and ADH activity (mean ± SD)**

| Test group (n=9) | Alcohol intake (g) | Alcohol concentration (%) | Acetaldehyde (µg/dl) | ADH |
|---|---|---|---|---|
| A | 28.1 ±1.21 | 0.010 ±0.010 | 222.3 ±85.8 | 15.00 ±3.93 |
| B | 0 | 0.004 ±0.003 | 32.2 ±35.6 | 15.55 ±3.41 |
| C | 26.4 ±1.56 | 0.015 ±0.007 | 82.6 ±36.2 | 16.80 ±2.40 |
| D | 27.1 ±1.17 | 0.003 ±0.003 | 39.8 ±44 | 17.98 ±2.97 |

| | | | | |
|---|---|---|---|---|
| (n=9, mean ± SD) * ADH: alcohol dehydrogenase (nmol NADH/min/mg protein) Superscripts indicated with other letters have shown significant difference between mean values (p<0.05). | | | | |

In Test Examples 1-5, alcohol was administered to white rats for a long period (10 weeks), and the alcohol degradation efficacies of the commercial product as a hangover relief drink and the inventive natural tea were examined. From the above reported results, it can be concluded the following;
1) Growth rates were somewhat higher in the control group but not statistically different between the treated groups. Hematocrit (Ht) values were higher in the natural tea-administered group than in the other groups but not statistically different between the treated groups. Hemoglobin contents were statistically different between the treated groups (p<0.05).
2) Triglyceride (TG) and HDL-cholesterol contents, and atherogenic indexes were significantly different between the treated groups (p<0.05).
   TG contents were the lowest for the natural tea-administered group whereas HDL-cholesterol contents were the highest for the natural tea-administered group.
3) Liver function factors (bilirubin, ALP, GOT and γ-GTP) were significantly different between the treated groups (p<0.05).
   Bilirubin contents were 3.54 mg/dl, the highest value, in the control group, and 1.21 mg/dl, the lowest value, in the inventive natural tea-administered group. ALP activities were 17.7 K-A unit, the highest value, in the alcohol-only group, and 11.9 K-A unit, the lowest value, in the inventive natural tea-administered group. GOT content were 77.4 IU/L, the highest value, in the alcohol-only group, and 60.7 IU/L, the lowest value, in the inventive natural tea-administered group. γ-GTP content, which are used to evaluate alcoholic fatty liver, were higher on the order of the commercial hangover relief drink-administered group, the alcohol-only group, the inventive natural tea-administered group and the control group.
4) Blood ethanol concentrations were 0.003%, the lowest value, for the natural tea-administered group (p<0.05), and blood acetaldehyde concentrations were 82.6 ± 36.2 µg/dl and 39.8 ± 44.0 µg/dl in the commercial hangover relief drink-administered group and the natural tea-administered group, respectively. Thus, blood acetaldehyde concentration was lower in the natural tea-administered group than in the commercial hangover relief drink-administered group. Meanwhile, alcohol dehydrogenase activities had no significance between the treated groups but were higher in the natural tea group than in the other groups.

To summarize, alcohol was administered orally to white rats for a long period (10 weeks) at an amount sufficient to cause alcoholic liver damage after which the inventive natural tea (Dawn-808), a hangover relief drink, was administered to the white rats from three weeks before the end of the test. In this case, the white rat group administered with the inventive natural tea showed excellent results for serum lipid concentration and liver functions as compared to the other groups. Furthermore, in the natural tea-administered group, ADH activity in liver tissue was somewhat higher than the other groups, and blood alcohol concentration was significantly lower than the other groups. This suggests that the inventive natural tea has the effect of promoting alcohol metabolism.

### Test Example 6: Change in blood alcohol and acetaldehyde concentrations

Tables 5 and 6 below show blood alcohol concentrations measured in first and second clinical tests, respectively.

In the first clinical test, test volunteers following an occupation were drunk with at least bottle of Soju (distilled liquor). Table 5 shows blood alcohol concentrations together with the acetaldehyde concentrations in blood drawn at 12 hours (9:00 AM the next day) after drinking alcohol. The blood alcohol concentrations measured with an alcolmeter were not statistically different between a test group and a control group, but were lower in the inventive natural tea-drunk test group than in the test group with the passage of time. The blood alcohol concentrations measured at 12 hours after drinking alcohol were 0.006% and 0.030% in the test group and the control group, respectively. Meanwhile, the blood acetaldehyde concentrations measured at the same time as the blood alcohol concentrations in the test group was reduced to 1/2 lower level than that in the control group. Such results generally coincide with the test group volunteer's answer that the inventive natural tea helped to relieve hangovers on the next day after drinking alcohol.

**Table 5: Effect of natural tea on blood alcohol and acetaldehyde concentrations**

| Volunteers (n=12) | Blood alcohol concentration (%), | | | | | Acetaldehyde (%, X 10⁻⁴) |
|---|---|---|---|---|---|---|
| | 0 min | 60 min | 120 min | 150 min | 720 min | 720 min |
| Test group | 0.148 ± 0.049 | 0.117 ± 0.065 | 0.098 ± 0.051 | 0.058 ± 0.055 | 0.006 ± 0.007 | 7.72 ± 6.00 |
| Control group | 0.173 ± 0.065 | 0.169 ± 0.081 | 0.140 ± 0.066 | 0.082 ± 0.034 | 0.030 ± 0.032 | 15.78 ± 3.94 |

| | | | | | | |
|---|---|---|---|---|---|---|
| (first clinical test) (mean ± SD) | | | | | | |

Table 6 shows the results of a second clinical test. In the second clinical test, university students drank one bottle of Passport®, a Korean whisky brand, and additional drinking of alcohol was prohibited. Blood alcohol concentrations were lower than those in the first clinical test where the test volunteers drank at least one bottle of soju, one kind of Korean alcohol beverage. Presumably, results of the second clinical test were as such because the amount of alcohol consumed was less and the age of the test volunteers was lower than the volunteers in the first clinical test.

Like the first clinical test, the test group administered with the inventive natural tea showed lower blood alcohol concentrations than the control group, except for a point 30 minutes after drinking alcohol. The blood alcohol concentrations at 360 minutes after drinking alcohol were 0.016% in the test group and 0.033% in the control group, indicating a great difference in blood alcohol concentrations between the two groups.

Meanwhile, blood acetaldehyde concentrations were measured 60 minutes, 150 minutes and 240 minutes after drinking alcohol, and the results are given in Table 7 below. The blood acetaldehyde concentration at 60 minutes after drinking alcohol had no significance between the two groups but was far lower in the test group than in the control group. This difference in blood alcohol concentration was reduced at 120 minutes and 240 minutes after drinking alcohol. The acetaldehyde concentration had a correlation with the blood alcohol concentration.

**Table 6: Effect of natural tea on blood alcohol concentration**

| Volunteers (n=14) | Blood alcohol concentration (%) | | | | | | |
|---|---|---|---|---|---|---|---|
| | 0 min | 30 min | 60 min | 120 min | 180 min | 270 min | 360 min |
| Test group | 0.107 ± 0.020 | 0.111 ± 0.049 | 0.087 ± 0.025 | 0.058 ± 0.037 | 0.042 ± 0.033 | 0.029 ± 0.030 | 0.016 ± 0.024 |
| Control group | 0.115 ± 0.013 | 0.107 ± 0.029 | 0.103 ± 0.013 | 0.101 ± 0.046 | 0.080 ± 0.042 | 0.057 ± 0.031 | 0.033 ± 0.026 |

| | | | | | | | |
|---|---|---|---|---|---|---|---|
| (second clinical test) (mean ± SD) | | | | | | | |

**Table 7: Effect of natural tea on blood acetaldehyde concentration**

| Volunteers (n=14) | Acetaldehyde (%, x 10⁻¹) | | |
|---|---|---|---|
| | 60 min | 150 min | 240 min |
| Test group | 78.6 ± 33.67 | 60.35 ± 25.81 | 33.12 ± 12.70 |
| Control group | 104.62 ± 45.93 | 77.69 ± 39.08 | 43.03 ± 10.94 |

| | | | |
|---|---|---|---|
| (second clinical test) (mean ± SD) | | | |

### Test Example 7: Change in liver function activity

Table 8 shows a change in liver function activity of blood collected in the second clinical test. GOT and GPT are numerical values for enzymes which are transferred from liver cells to blood when the liver cells are broken due to inflammation in the liver. GOT which indicates the degree of breakdown of the liver cells was lower in the test group than in the control group with the passage of time. Particularly at 150 minutes after drinking alcohol, GOT was statistically significantly lower in the test group than in the control group (p<0.05).

γ-GTP, which is used to evaluate fatty liver and alcoholic liver diseases, was higher in the test group than in the control group at 0 minutes after drinking alcohol. However, γ-GTP in the test group was then reduced and reached a similar value to that in the control group at 240 minutes after drinking alcohol.

**Table 8: Effect of natural tea on liver functions**

| (mean ± SD) | | | | | |
|---|---|---|---|---|---|
| Measurement items | | Liver function activity | | | |
| | | 0 min | 60 min | 150 min | 240 min |
| GOT | Test group | 63.4 ± 12.4 | 68.2 ± 12.6 | 41.8 ± 7.00** | 31.2 ± 7.3 |
| | Control group | 62.0 ± 7.5 | 89.4 ± 16.4 | 89.0 ± 9.4 | 40.1 ± 4.8 |
| GPT | Test group | 36.8 ± 5.9 | 49.6 ± 10.6 | 41.4 ± 12.4 | 24.1 ± 7.5 |
| | Control group | 45.6 ± 6.0 | 57.3 ± 11.6 | 56.7 ± 9.4 | 33.1 ± 4.5 |
| γ-GTP | Test group | 45.9 ± 10.2 | 35.9 ± 3.1 | 32.4 ± 3.2 | 27.4 ± 8.5 |
| | Control group | 34.7 ± 3.0 | 60.6 ± 16.6 | 58.4 ± 12.7 | 28.6 ± 1.9 |

| | | | | | |
|---|---|---|---|---|---|
| ** (p<0.05) GOT & GPT (Kamen unit) γ-GTP (mU/ml) | | | | | |

### Test Example 8: Change in serum lipid

Table 9 below shows serum triglyceride (TG) and total cholesterol (TC) contents.

Blood TG content was lower in the test group than in the control group at all the time point of measurements. This seems attributable to a difference in TG content between the two groups upon the start of measurement. However, the serum TG content in the control group reached the highest value of 354.3 mg/dl at 150 minutes after drinking alcohol and then was reduced by about 100 mg/dl at 240 minutes after drinking. In both the two groups, TG contents were higher at 240 minutes than at 0 minute. Blood TG contents were reduced in both the two groups with the passage of time after drinking alcohol.

**Table 9: Effect of natural tea on serum lipid content**

| (mean ± DS) | | | | | |
|---|---|---|---|---|---|
| Measurement items | | Serum lipid (mg/dl) | | | |
| | | 0 min | 60 min | 150 min | 240 min |
| TG | Test group | 204.4 ± 42.8 | 229.0 ± 34.0 | 226.2 ± 32.5 | 224.8 ± 57.0 |
| | Control group | 233.8 ± 49.1 | 261.8 ± 55.3 | 354.3 ± 56.2 | 255.2 ± 33.7 |
| TC | Test group | 162.5 ± 6.7 | 166.3 ± 8.8 | 160.1 ± 9.8 | 142.2 ± 10.0 |
| | Control group | 188.5 ± 12.6 | 177.2 ± 13.0 | 183.8 ± 14.3 | 134.5 ± 8.3 |

| | | | | | |
|---|---|---|---|---|---|
| TG: triglyceride TC: total cholesterol | | | | | |

### Test Example 9: Change in serum ADH activity

Alcohol dehydrogenase activity having a determinative effect on alcohol degradation follows zero order reaction where alcohol is metabolized only at a constant amount per unit time regardless of the alcohol concentration in cells. Thus, the activity of this enzyme is constant regardless of the degree of drinking (Takagi et al., 1985; and Oshii et al., 1973).

Table 10 below shows a change in ADH activity with time.

The ADH activities measured at 0 min. after drinking alcohol were 2.63 U/L and 3.53 U/L in the test group and the control group, respectively, indicating that the ADH activity in the control group was higher than the test group but had no significant statistical difference from the test group. However, regarding a change in ADH activity with time, the test group showed an ADH activity of 4.12 U/L at 60 minute after drinking alcohol, which indicates more than 50% increase in ADH activity as compared to 2.63 U/L at 0 minute. Also, the test group showed an ADH activity of 3.76 U/L at 150 minutes and an ADH activity of 3.09 U/L at 240 minutes, which is about 17% higher than the ADH activity at 0 minute after drinking alcohol. On the other hand, in the control group, the measured ADH activity values at 150 minutes and 240 minutes were somewhat inconstant, but the ADH activity at 0 minutes after drinking was reduced with the passage of time and reduced by more than 20% at 240 minutes after drinking alcohol.

**Table 10: Effect of natural tea on ADH activity**

| (mean ± SD) | | | | |
|---|---|---|---|---|
| Groups (n=14) | ADH activity (U/L) | | | |
| | 0 min | 60 min | 150 min | 240 min |
| Test group | 2.63 ± 0.42 | 4.12 ± 1_{.}43 | 3.76 ± 0.83 | 3.09 ± 0.89 |
| Control group | 3.53 ± 0.83 | 3.49 ± 0.77 | 2.67 ± 0.63 | 2.76 ± 0.64 |

As can be seen in Table 10, although there was no statistical difference between the two groups, the test group which ingested the inventive natural tea showed a somewhat higher ADH activity than the control group. This suggests that the inventive natural tea promoted blood alcohol metabolism.

In Test Examples 6-9 above, the test volunteers were administered with the inventive natural tea before or after drinking alcohol, and measured for their alcohol degradation ability in a crossover experimental design. The test results provide the following possible conclusions.
1) Blood alcohol concentrations at all the time points of measurement were lower in the inventive natural tea-administered test group than in the control group but had no statistical significance between the two groups. Also, blood acetaldehyde concentrations were lower in test group than in the control group.
2) Regarding liver function activity, GOT concentration became lower in the test group than in the control group with the passage of time. Particularly, at 150 minutes after drinking, there came to be a statistical significance between the two groups (p<0.05).
   GPT and γ-GTP concentrations reached the highest values at 60 minutes after drinking and then declined. GPT and γ-GTP concentrations were somewhat lower in the natural tea-administered test group than in the control group.
3) Regarding serum lipid content, triglyceride (TG) content was higher in the control group than in the test group. Particularly in the control group, TG content reached the highest value at 150 minutes and then declined by about 100 mg/dl at 240 minutes. In both the two groups, the TG content at 240 minutes was higher than at 0 minutes.
   Total cholesterol (TC) contents were reduced in both the two groups with the passage of time after drinking alcohol.
4) Meanwhile, in a question survey conducted on the volunteers who participated in the clinical tests, the respondents answered that hangover syndromes, such as headache, heartburn and asthenia, which are usually experienced on the next day after drinking alcohol, were relieved after taking the inventive natural tea.

As described above, the present invention provides the natural powder or liquid tea comprising an extract from *Robinia pseudo-acacia,* and optionally, a given amount of extracts from *Hedyotis diffusa, Amomi semen, Glycyrrhiza* and/or *Pueraiae Flos,* as well as the method for preparing the same. The natural tea according to the present invention shows the effects of relieving hangovers and protecting the liver when taken before or after drinking alcohol. Particularly, according to the present invention, robinine extracted from *Robinia pseudo-acacia* can be used as a single active ingredient in a hangover relief drink.

## Claims

1. Natural tea including an extract from *Robinia pseudo-acacia* as a main active ingredient and a conventional additive selected from sweetening agents, spices and colouring agents, obtainable by a method comprising the steps of:
washing the leaf, stem, flower and root of *Robinia pseudo-acacia* with water;
extracting the water-washed material with an alcohol extraction solvent at 50-150°C for 1-10 hours to give an extract to be used as an active ingredient; and
adding a conventional additive selected from sweetening agents, spices and colouring agents to the extract;
for use in revitalising liver functions.

2. A natural tea as claimed in claim 1, including a *Hedyotis diffusa* extract, an *Amomi semen* extract, a *Glycyrrhiza* extract and/or a *Pueraiae Flos* extract, as a minor active ingredient.

3. A natural tea as claimed in claim 1 or 2, which comprises a mixture of 10-80% by weight of the *Robina pseudo-acacia* extract and 20-90% by weight of a *Hedyotis diffusa* extract.

4. A natural tea as claimed in claim 2, which comprises a mixture of (i) 10-65% by weight of the *Robinia pseudo-acacia* extract, (ii) 20-80% by weight of the *Hedyotis diffusa* extract, and (iii) 10-60% by weight of the *Amomi semen* extract and/or 10-50% by weight of the *Glycyrrhiza* extract.

5. A natural tea as claimed in claim 2, obtainable by extracting 10-30% by weight of *Robinia pseudo-acacia,* 20-60% by weight of *Hedyotis diffusa,* 10-40% by weight of *Amomi semen,* 10-20% by weight of *Glycyrrhiza* and 10-30% by weight of *Pueraiae Flos,* with an extraction solvent.

6. A method for preparing natural tea as claimed in any preceding claim, comprising the steps of:
washing the leaf, stem, flower and root of *Robinia pseudo-acacia* with water;
extracting the water-washed material with an alcohol extraction solvent at 50-150°C for 1-10 hours to give an extract to be used as an active ingredient; and
adding a conventional additive selected from sweetening agents, spices and colouring agents to the extract.

7. A method as claimed in claim 6, which additionally comprises adding at least one minor active ingredient comprising *Hedyotis diffusa* extract, *Amomi semen* extract, *Glycyrrhiza* extract and/or *Pueraiae Flos* extract, to the *Robinia pseudo-acacia* extract.

8. A method as claimed in claim 6 or 7, wherein 10-80% by weight of the *Robinia pseudo-acacia* extract and 20-90% by weight of a *Hedyotis diffusa* extract are mixed with each other.

9. A method as claimed in claim 8, wherein (i) 10-65% by weight of the *Robinia pseudo-acacia* extract, (ii) 20-80% by weight of the *Hedyotis diffusa* extract, and (iii) 10-60% by weight of the *Amomi semen* extract and/or 10-50% by weight of the *Glycyrrhiza* extract are mixed with other.

10. A method as claimed in claim 7, wherein 10-30% by weight of *Hedyotis diffusa,* 10-40% by weight of *Amomi semen,* 10-20% by weight of *Glycyrrhiza* and 10-30% by weight of *Pueraiae Flos* are mixed and then extracted with the extraction solvent.

11. A method as claimed in any of claims 6 to 9, which additionally comprises centrifuging the *Robinia pseudo-acacia* extract at 2,500-7,500 rpm to separate robinine for use.

12. A method as claimed in any of claims 6 to 11, additionally comprising centrifuging the extracts at 5000-7,500 rpm to separate active ingredients for use.

## Patentansprüche

1. Natürlicher Tee enthaltend einen Extrakt aus *Robinia pseudo-acacia* als einem Hauptwirkstoff und einem herkömmlichen Zusatzstoff ausgewählt aus Süßungsmitteln, Gewürzen und Farbstoffen, erhältlich mittels eines Verfahrens mit folgenden Schritten:
Waschen des Blattes, des Stieles, der Blüte und der Wurzel von *Robinia pseudo-acacia* mit Wasser;
Extrahieren des mit Wasser gewaschenen Materials mit einem alkoholischen Extraktionslösungsmittel bei 50 bis 150 °C für 1 bis 10 Stunden, um einen als Wirkstoff zu verwendenden Extrakt zu ergeben; und
Hinzufügen eines herkömmlichen Zusatzstoffes ausgewählt aus Süßungsmitteln, Gewürzen und Farbstoffen zu dem Extrakt;
zur Verwendung für die Revitalisierung von Leberfunktionen.

2. Natürlicher Tee nach Anspruch 1,
einschließend einen Extrakt aus *Hedyotis diffusa,* einen Extrakt aus *Amomi semen,* einen Extrakt aus *Glycyrrhiza* und/oder einen Extrakt aus *Pueraiae Flos* als Nebenwirkstoff.

3. Natürlicher Tee nach Anspruch 1 oder 2,
welcher eine Mischung aus 10 bis 80 Gewichtsprozent von einem Extrakt aus *Robinia pseudo-acacia* und 20 bis 90 Gewichtsprozent eines Extrakts aus *Hedyotis diffusa* aufweist.

4. Natürlicher Tee nach Anspruch 2,
welcher eine Mischung aus (i) 10 bis 65 Gewichtsprozent eines Extrakts aus *Robinia pseudo-acacia,* (ii) 20 bis 80 Gewichtsprozent eines Extrakts aus *Hedyotis diffusa,* und (iii) 10 bis 60 Gewichtsprozent eines Extrakts aus *Amomi semen* und/oder 10 bis 50 Gewichtsprozent eines Extrakts aus *Glycyrrhiza* aufweist.

5. Natürlicher Tee nach Anspruch 2,
erhältlich durch das Extrahieren von 10 bis 30 Gewichtsprozent von *Robinia pseudo-acacia,* 20 bis 60 Gewichtsprozent von *Hedyotis diffusa,* 10 bis 40 Gewichtsprozent von *Amomi semen,* 10 bis 20 Gewichtsprozent von *Glycyrrhiza* und 10 bis 30 Gewichtsprozent von *Pueraiae Flos* mittels eines Extraktionslösungsmittels.

6. Verfahren zum Vorbereiten von natürlichem Tee nach einem der vorstehenden Ansprüche,
aufweisend folgende Schritte:
Waschen des Blattes, des Stieles, der Blüte und der Wurzel von *Robinia pseudo-acacia* mit Wasser;
Extrahieren des mit Wasser gewaschenen Materials mit einem alkoholischen Extraktionslösungsmittel bei 50 bis 150 °C für 1 bis 10 Stunden, um einen als Wirkstoff zu verwendenden Extrakt zu ergeben; und
Hinzufügen eines herkömmlichen Zusatzstoffes ausgewählt aus Süßungsmitteln, Gewürzen und Farbstoffen zu dem Extrakt.

7. Verfahren nach Anspruch 6,
welches außerdem das Hinzufügen von zumindest einem Nebenwirkstoff aufweisend einen Extrakt aus *Hedyotis diffusa,* einen Extrakt aus *Amomi semen,* einen Extrakt aus *Glycyrrhiza* und/oder einen Extrakt aus *Pueraiae Flos* zu dem Extrakt aus *Robinia pseudo-acacia* aufweist.

8. Verfahren nach Anspruch 6 oder 7,
in welchem 10 bis 80 Gewichtsprozent eines Extrakts aus *Robinia pseudo-acacia* und 20 bis 90 Gewichtsprozent eines Extrakts aus *Hedyotis diffusa* miteinander gemischt werden.

9. Verfahren nach Anspruch 8,
in welchem (i) 10 bis 65 Gewichtsprozent eines Extrakts aus *Robinia pseudo-acacia,* (ii) 20 bis 80 Gewichtsprozent eines Extrakts aus *Hedyotis diffusa,* und (iii) 10 bis 60 Gewichtsprozent eines Extrakts aus *Amomi semen* und/oder 10 bis 50 Gewichtsprozent eines Extrakts aus *Glycyrrhiza* miteinander gemischt werden.

10. Verfahren nach Anspruch 7,
in welchem 10 bis 30 Gewichtsprozent von *Hedyotis diffusa,* 10 bis 40 Gewichtsprozent von *Amomi semen,* 10 bis 20 Gewichtsprozent von *Glycyrrhiza* und 10 bis 30 Gewichtsprozent von *Pueraiae Flos* gemischt und dann mittels des Extraktionslösungsmittels extrahiert werden.

11. Verfahren nach einem der Ansprüche 6 bis 9,
welches zusätzlich das Zentrifugieren des Extraktes aus *Robinia pseudo-acacia* mit 2500 bis 7500 Umdrehungen / Minute aufweist, um Robinin zur Verwendung zu separieren.

12. Verfahren nach einem der Ansprüche 6 bis 11,
zusätzlich aufweisend das Zentrifugieren der Extrakte bei 5000 bis 7500 Umdrehungen / Minute zum Separieren von Wirkstoffen zur Verwendung.

## Revendications

1. Thé naturel comprenant un extrait de *Robinia pseudoacacia* comme principe actif principal et un additif classique choisi parmi les agents édulcorants, les épices et les agents colorants, pouvant être obtenu par un procédé comprenant les étapes consistant à :
laver la feuille, la tige, la fleur et la racine de *Robinia pseudoacacia* avec de l'eau ;
extraire la matière lavée à l'eau avec un solvant d'extraction alcoolique à 50-150 °C pendant 1-10 heures pour donner un extrait destiné à être utilisé comme principe actif ; et
ajouter à l'extrait un additif classique choisi parmi les agents édulcorants, les épices et les agents colorants ;
destiné à être utilisé pour relancer des fonctions hépatiques.

2. Thé naturel selon la revendication 1, comprenant un extrait de *Hedyotis diffusa,* un extrait *d'Amomi semen,* un extrait de *Glycyrrhiza* et/ou un extrait de *Pueraiae flos,* comme principe actif secondaire.

3. Thé naturel selon la revendication 1 ou 2, qui comprend un mélange de 10-80 % en poids de l'extrait de *Robinia pseudoacacia* et de 20-90 % en poids d'un extrait de *Hedyotis diffusa.*

4. Thé naturel selon la revendication 2, qui comprend un mélange de (i) 10-65 % en poids de l'extrait de *Robinia pseudoacacia,* (ii) 20-80 % en poids de l'extrait de *Hedyotis diffusa* et (iii) 10-60 % en poids de l'extrait *d'Amomi semen* et/ou 10-50 % en poids de l'extrait de *Glycyrrhiza.*

5. Thé naturel selon la revendication 2, pouvant être obtenu par extraction de 10-30 % en poids de *Robinia pseudoacacia,* 20-60 % en poids de *Hedyotis diffusa,* 10-40 % en poids *d'Amomi semen,* 10-20 % en poids de *Glycyrrhiza* et 10-30 % en poids de *Pueraiae flos,* avec un solvant d'extraction.

6. Procédé pour la préparation de thé naturel selon l'une quelconque des revendications précédentes, comprenant les étapes consistant à :
laver la feuille, la tige, la fleur et la racine de *Robinia pseudoacacia* avec de l'eau ;
extraire la matière lavée à l'eau avec un solvant d'extraction alcoolique à 50-150 °C pendant 1-10 heures pour donner un extrait destiné à être utilisé comme principe actif ; et
ajouter à l'extrait un additif classique choisi parmi les agents édulcorants, les épices et les agents colorants.

7. Procédé selon la revendication 6, qui comprend de plus l'ajout à l'extrait de *Robinia pseudoacacia* d'au moins un principe actif secondaire comprenant de l'extrait de *Hedyotis diffusa,* de l'extrait d'*Amomi semen,* de l'extrait de *Glycyrrhiza* et/ou de l'extrait de *Pueraiae flos.*

8. Procédé selon la revendication 6 ou 7, dans lequel 10-80 % en poids de l'extrait de *Robinia pseudoacacia* et 20-90 % en poids d'un extrait de *Hedyotis diffusa* sont mélangés l'un avec l'autre.

9. Procédé selon la revendication 8, dans lequel (i) 10-65 % en poids de l'extrait de *Robinia pseudoacacia,* (ii) 20-80 % en poids de l'extrait de *Hedyotis diffusa* et (iii) 10-60 % en poids de l'extrait *d'Amomi semen* et/ou 10-50 % en poids de l'extrait de *Glycyrrhiza* sont mélangés les uns avec les autres.

10. Procédé selon la revendication 7, dans lequel 10-30 % en poids de *Hedyotis diffusa,* 10-40 % en poids *d'Amomi semen,* 10-20 % en poids de *Glycyrrhiza* et 10-30 % en poids de *Pueraiae flos* sont mélangés et ensuite extraits avec le solvant d'extraction.

11. Procédé selon l'une quelconque des revendications 6 à 9, qui comprend de plus la centrifugation de l'extrait de *Robinia pseudoacacia* à 2 500-7 500 tours/min pour séparer la robinine destinée à être utilisée.

12. Procédé selon l'une quelconque des revendications 6 à 11, comprenant de plus la centrifugation des extraits à 5 000-7 500 tours/min pour séparer les principes actifs destinés à être utilisés.
